(19) Europäisches Patentamt
European Patent Office
Office européen des brevets

(11) Publication number: **0 107 496**
**B1**

# (12) EUROPEAN PATENT SPECIFICATION

(45) Date of publication of patent specification: **04.12.85**

(51) Int. Cl.[4]: **C 07 C 69/14,** C 07 C 67/297

(21) Application number: **83306417.3**

(22) Date of filing: **21.10.83**

## (54) Process for the preparation of esters.

(30) Priority: **27.10.82 GB 8230689**

(43) Date of publication of application:
**02.05.84 Bulletin 84/18**

(45) Publication of the grant of the patent:
**04.12.85 Bulletin 85/49**

(84) Designated Contracting States:
**BE DE FR GB IT NL**

(56) References cited:
**EP-A-0 034 062**
**EP-A-0 061 395**

(73) Proprietor: **BP Chemicals Limited**
**Belgrave House 76 Buckingham Palace Road**
**London, SW1W 0SU (GB)**

(72) Inventor: **Drury, David James**
**BP Chemicals Limited**
**Belgrave House 76 Buckingham Palace Road**
**London, SW1W 0SU (GB)**
Inventor: **Kent, Alexander George**
**BP Chemicals Limited**
**Saltend**
**Hedon Hull, HU12 8DS (GB)**

(74) Representative: **Crack, Richard David et al**
**c/o The British Petroleum Company plc Patents Division Chertsey Road**
**Sunbury-on-Thames Middlesex, TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 107 496 B1

## Description

This invention relates to a process for the production of esters, more particularly to the production of esters of acetic acid, such as ethyl acetate.

Ethyl acetate is a valuable organic chemical and is used as a solvent, for example, in printing inks, adhesives, lacquers and as a solvent for cellulose nitrate and as an organic intermediate (see Kirk-Othmer "Encyclopaedia of Chemical Technology" 3rd Edition, Volume 9, page 329, Interscience New York 1980).

It has been previously proposed to prepare ethyl acetate from methyl acetate in, for example, European Patent 0031606 and *J. Amer, Chem. Soc. 100* 6238. The methods proposed however suffer from the disadvantage of slow reaction rates and forcing conditions being required. Other methods from $C_1$ starting materials have been proposed but require two or more step processes. Ethylidene diacetate is, however, readily available from methyl acetate by a fast reaction or from acetic anhydride and acetaldehyde, and it is therefore an object of the present invention to provide a process for converting ethylidene diacetate to ethyl acetate.

The reaction of acetic anhydride with hydrogen to form ethylidene diacetate and ethylacetate using a homogeneous catalyst which is a combination of (i) a rhodium or ruthenium compound with methyl iodide and lithium iodide or (2) a ruthenium compound with methyl iodide, has been previously described in Patent Application WO 82/02712. In one embodiment of the process described, the starting material can be a mixture of acetic anhydride and ethylidene diacetate and is hydrogenated to form a mixture of ethyl acetate and acetic acid.

It has now been found that the hydrogenation of ethylidene diacetate to form ethyl acetate can be significantly improved by employing, as the starting material, ethylidene diacetate which is substantially free of acetic anhydride or contains an amount of acetaldehyde that is at least equimolar with respect to the anhydride.

According to the present invention a process for the preparation of a carboxylic acid ester of formula $R^1COOR^2$ where $R^1$ and $R^2$ which may be the same or different, are monovalent hydrocarbyl radicals comprises reacting a diester compound of formula:

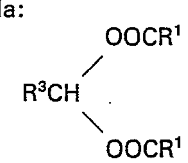

where $R^1$ is as defined above and $R^3$ is also a monovalent hydrocarbyl radical with hydrogen in the presence of, as catalyst, an effective amount of a Group VIII metal compound the diester either being substantially free of anhydride of the formula $(R^1CO)_2O$ or containing an amount of aldehyde of formula $R^3CHO$ providing a molar ratio of aldehyde to anhydride of at least 1:1.

Conveniently the radicals $R^1$, $R^2$ and $R^3$ are $C_1$ to $C_{12}$ radicals.

The above process is particularly suitable for the preparation of ethyl acetate from ethylidene diacetate, a by product of which reaction is acetic acid. Under certain conditions the valuable chemical diethyl ether may also be formed.

In the case of ethylidene diacetate the process is believed to involve the following steps

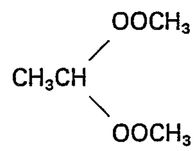

$\rightleftharpoons CH_3CHO + (CH_3CO)_2O$ (I)

$$CH_3CHO + H_2 \rightarrow CH_3CH_2OH \quad (II)$$

$$CH_3CH_2OH + (CH_3CO)_2O \rightarrow CH_3COOCH_3CH_3 + CH_3COOH \quad (III)$$

Overall

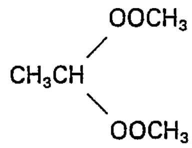

$+ H_2 \rightarrow CH_3CO_2CH_2CH_3 + CH_3COOH$

It will be seen from the equation (I) that the presence of acetic anhydride in an amount greater than that formed by equation (I) will reduce the acetaldehyde concentration and hence the rate of formation of ethyl acetate. It is therefore advantageous to use an ethylidene diacetate feed that is free from acetic anhydride.

The Patent Application WO 82/02712 referred to above, so far as it mentions ethylidene diacetate as a reactant, discloses only its reaction with hydrogen. According to the present invention it has been found that the presence of carbon monoxide is beneficial in that it improves the reaction rate. Conveniently the amount of carbon monoxide is at least 1%.

The proportion of carbon monoxide is preferably greater than 5 vol % more preferably from 10 to 35 vol% based on the volume of the hydrogen and other reactants and solvent (if present).

When preparing ethyl acetate from ethylidene diacetate the process is conveniently effected from reactants which initially consist substantially of ethylidene diacetate, hydrogen and carbon monoxide, if present, for example, the liquid phase reactant may contain at least 95% by weight of ethylidene diacetate. The process can be effected by feeding reactants comprising ethylidene diacetate, carbon monoxide and hydrogen to a reaction zone.

The ethylidene diacetate can be prepared from acetaldehyde and acetic anhydride in a separate step or can be formed in situ, employing a molar ratio of acetaldehyde to acetic anhydride of at least 1:1. Thus a mixture of acetaldehyde and acetic anhydride can be passed to the reaction zone.

Conveniently the reaction is effected at elevated temperature under conditions that maintain the diester reactant and the ester product in the liquid phase. Suitable temperatures are, for example, up to 200°C preferably in the range 50 to 180°C.

Conveniently the combined pressure of hydrogen and carbon monoxide is in the range 30 to 120 bar at the reaction temperature.

By the term Group VIII metal is meant iron, cobalt, nickel, ruthenium, rhodium, palladium, osmium, iridium and platinum.

Conveniently the Group VIII metal compound is a ruthenium, iridium or cobalt compound which is conveniently present in a form that is soluble in the liquid phase reactants and products for example, a salt or carbonyl compound.

Conveniently concentrations of catalyst are from 100 ppm upwards for example up to 100,000 ppm preferably in the range 100 to 50,000 by weight based on the weight of diester reactant.

Preferably the catalyst system is substantially iodide-free, although iodide may be present for example, as the Group VIII metal iodide.

A solvent for the reactants and products can be employed, for example ethylidene diacetate/acetic acid mixtures.

When diester is the starting material the term substantially free of anhydride is intended to mean that the diester contains less than 5% by wt of the anhydride. However since the diester can be replaced by its precursors, for example, acetaldehyde and acetic anhydride can be used instead of ethylidene diacetate, the term means that where acetic anhydride is present then there is also present at least an equivalent amount (1 molecule for 1 molecule) of acetaldehyde.

It is therefore within the scope of the present invention to employ as feed a mixture of ethylidene diacetate and its precursors mentioned above provided that the molar ratio of acetaldehyde to acetic anhydride is at least 1:1.

Desirably the reaction mixture is substantially anhydrous.

The invention is illustrated by the following Examples.

In all the examples, the reactants and products (with the exception of the carbon monoxide and hydrogen) were maintained in the liquid phase and the catalyst was used in solution.

The ethylidene diacetate and the other reactants were pure and contained no acetic anhydride. All reactants were anhydrous.

Example 1

Conversion of ethylidene diacetate to ethyl acetate, acetic acid and diethyl ether.

A 100 ml Hastelloy 'B2' autoclave was charged with 25.0 g ethylidene diacetate and 0.202 g ruthenium trichloride and pressurized with carbon monoxide (200 psi) and hydrogen (1000 psi). The temperature was brought to 150°C and the reaction continued for 45 mins with stirring. After cooling, gas chromatography (g.c.) analysis of the discharge showed it to contain 8.5 g ethyl acetate, 11.9 g of acetic acid and 2.0 g diethyl ether.

Example 2

Use of iridium catalyst

A 100 ml Hastelloy 'B2' autoclave was charged with 0.105 g iridium trichloride and 25.0 g ethylidene diacetate and pressurised with hydrogen and carbon monoxide (respective partial pressures 1000 and 200 psi). The autoclave was brought to 180°C and the reaction continued for $2\frac{1}{2}$ hours with stirring. After cooling, the discharge was analysed by g.c. and shown to contain 3.3 g ethyl acetate and 6.7 g acetic acid.

Example 3

Use of cobalt catalyst

A 100 ml autoclave was charged with 0.200 g dicobalt octacarbonyl and 30.0 g ethylidene diacetate and pressurised with hydrogen and carbon monoxide (respective partial pressures 1000 and 200 psi). The autoclave was brought to 200°C and the reaction continued for 2 hours with stirring. After cooling, the discharge was analysed by g.c. and shown to contain 1.2 g ethyl acetate and 8.0 g acetic acid.

Example 4

Reaction in the absence of carbon monoxide

A 100 ml Hastelloy 'B2' autoclave was charged with 0.103 g ruthenium trichloride and 25.0 g ethylidene diacetate and pressurised with hydrogen to 1000 psi. The autoclave was brought to 150°C and the reaction continued for $2\frac{1}{2}$ hours with stirring. After cooling, the discharge was analysed by g.c. and shown to contain 2.6 g ethyl acetate and 4.9 g acetic acid.

The advantages of the iodide free catalyst system described is that the necessity of using iodide corrosion resistant materials and iodide toxicity problems are avoided.

Example 5

A 100 ml Hastelloy B2 autoclave was charged with 0.205 g of ruthenium trichloride and 25.0 g of propylidene diacetate and pressurised with hydrogen and carbon monoxide (respective partial pressures 1000 and 200 psi). The autoclave was brought to 180°C and the reaction continued for $1\frac{1}{4}$ hours with stirring. After cooling the discharge was analysed by gc and shown to contain a propyl acetate, di n propyl ether and acetic acid.

**Claims**

1. A process for the preparation of a carboxylic acid ester of formula $R^1COOR^2$ where $R^1$ and $R^2$ which may be the same or different, are monovalent hydrocarbyl radicals which process comprises reacting a diester compound of formula:

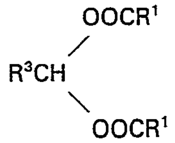

where $R^1$ is as defined above and $R^3$ is also a monovalent hydrocarbyl radical with hydrogen in the presence of, as catalyst, an effective amount of a Group VIII metal compound the diester either being substantially free of anhydride of the formula $(R^1CO)_2O$ or containing an amount of aldehyde of formula $R^3CHO$ providing a molar ratio of aldehyde to anhydride of at least 1:1.

2. A process as claimed in claim 1 which comprises carrying out the process in the presence of carbon monoxide.

3. A process as claimed in claim 1 or 2 wherein the group VIII metal is a noble metal.

4. A process as claimed in claim 3 wherein the noble metal is iridium or ruthenium.

5. A process as claimed in claim 1 or 2 wherein the Group VIII metal is cobalt.

6. A process as claimed in any one of the preceding claims wherein the reaction conditions are such as to maintain the diester reactant and the ester product in the liquid phase.

7. A process as claimed in any one of the preceding claims wherein a solvent for the diester reactant, catalyst and ester product is employed.

8. A process as claimed in any one of the preceding claims wherein the diester is ethylidene diacetate and is formed in situ from acetaldehyde and acetic anhydride in a molar ratio of at least 1:1.

**Patentansprüche**

1. Verfahren zur Herstellung von Carbonsäureestern der Formel $R^1COOR^2$, worin $R^1$ und $R^2$ gleich oder verschieden sein können und einwertige Kohlenwasserstoffreste darstellen, durch Umsetzung eines Diesters der Formel

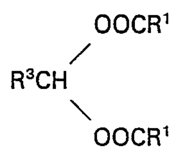

worin $R^1$ obige Bedeutung hat und $R^3$ ebenfalls ein einwertiger Kohlenwasserstoffrest ist, mit Wasserstoff in Anwesenheit einer wirksamen Menge einer Verbindung eines Metalles der Gruppe VIII als Katalysator, wobei der Diester entweder im wesentlichen frei von Anhydrid der Formel $(R^1CO)_2O$ ist oder eine Menge eines Aldehydes der Formel $R^3CHO$ enthält, so daß ein molares Verhältnis von Aldehyd zu Anhydrid von mindestens 1:1 gegeben ist.

2. Verfahren gemäß Anspruch 1, wobei das Verfahren in Anwesenheit von Kohlenmonoxid durchgeführt wird.

3. Verfahren gemäß Anspruch 1 oder 2, wobei das Metall der Gruppe VIII ein Edelmetall ist.

4. Verfahren gemäß Anspruch 3, wobei das Edelmetall Iridium oder Ruthenium ist.

5. Verfahren gemäß Anspruch 1 oder 2, wobei das Metall der Gruppe VIII Kobalt ist.

6. Verfahren gemäß einem der vorgehenden Ansprüche, wobei die Reaktionsbedingungen derart sind, daß der Diester-Ausgangsstoff und das Ester-Produkt in flüssiger Phase vorliegen.

7. Verfahren gemäß einem der vorgehenden Ansprüche, wobei ein Lösungsmittel für den Diester-Ausgangsstoff, den Katalysator und das Ester-Produkt eingesetzt wird.

8. Verfahren gemäß einem der vorgehenden Ansprüche, wobei der Diester Ethylidendiacetat und in situ gebildet wird aus Acetaldehyd und Essigsäureanhydrid in einem molaren Verhältnis von mindestens 1:1.

**Revendications**

1. Procédé de préparation d'un ester d'acide carboxylique de formule $R^1COOR^2$, dans laquelle $R^1$ et $R^2$, qui peuvent être identiques ou différents, sont des radicaux hydrocarbyles monovalents, ce procédé comprenant la réaction d'un diester de formule:

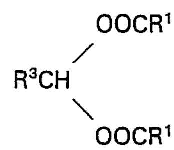

(dans laquelle $R^1$ est comme défini ci-dessus et $R^3$ est aussi un radical hydrocarbyle monovalent) avec l'hydrogène en présence, comme catalyseur, d'une quantité efficace d'un composé de métal du groupe VIII, le diester étant essentiellement dépourvu d'anhydride de formule $(R^1CO)_2O$ ou bien contenant une quantité d'aldéhyde de formule $R^3CHO$ fournissant un rapport molaire de l'aldéhyde à l'anhydride au moins égal à 1:1.

2. Procédé selon la revendication 1, qui comprend la mise en oeuvre du procédé en présence de monoxyde de carbone.

3. Procédé selon la revendication 1 ou 2, dans lequel le métal de groupe VIII est un métal noble.

4. Procédé selon la revendication 3, dans lequel le métal noble est l'iridium ou le ruthénium.

5. Procédé selon la revendication 1 ou 2, dans lequel le métal du groupe VIII est le cobalt.

6. Procédé selon l'une quelconque des revendications précédentes, dans lequel les conditions de réaction sont telles que l'on maintient en phase liquide le diester destiné à réagir et l'ester produit.

7. Procédé selon l'une quelconque des revendications précédentes, dans lequel on utilise un solvant pour le diester destiné à réagir, le catalyseur et l'ester produit.

8. Procédé selon l'une quelconque des revendications précédentes, dans lequel le diester est le diacétate d'éthylidène et il est formé sur place à partir d'acétaldéhyde et d'anhydride acétique présents en un rapport molaire d'au moins 1:1.